# EUROPEAN PATENT APPLICATION

(11) **EP 2 080 770 A1**
(43) Date of publication of application: **22.07.2009**
(21) Application number: 08100707.2
(22) Date of filing: 21.01.2008
(51) Int. Cl.: C07K 14/435, C07K 16/46

(54) **Proteinaceous binding molecules comprising purification tags**

(71) Applicant: MorphoSys AG, 82152 Martinsried/München (DE)
(72) Inventor: Steidl, Stefan, 81241 München (DE)
(74) Representative: Hutter, Bernd

(57) **Abstract**

The present invention provides proteinaceous binding molecules, in particular antibodies, having at least two variable domains. One variable domain binds to a target molecule of interest. At least one other variable domain comprises a purification tag suitable for purification of said proteinaceous binding molecule. The purification tag enables the convenient purification of the proteinaceous binding molecule with monovalent binding properties against the target molecule.

## Description

### Background of the invention

Immunoglobulins, such as antibodies, are of continued and increasing interest for the pharmaceutical industry. More than 20 monoclonal antibodies (mAb's) are on the market and more than 100 in clinical trials. In the last decade, numerous derivatives and fragments of immunoglobulins were designed that have certain different properties compared to conventional immunoglobulins and antibodies, which may be desirable for certain diagnostic and therapeutic applications. Examples are recombinant antibody fragments (such as classic monovalent antibody fragments (Fab, scFv)) or engineered variants (diabodies, triabodies, minibodies and single-domain antibodies), some of which are now emerging as credible alternatives. Such fragments and variants usually retain the target specificity of full-length immunoglobulins but can often be produced more economically. In addition, new 'antibody-like' protein display scaffolds were developed, which, likewise, possess certain other properties compared to conventional immunoglobulins and antibodies.

For certain therapeutic applications, it would desirable to have a monovalent antibody-antigen interaction, i.e. an immunoglobulin, or another scaffold, with only one variable domain that binds to a target molecule of interest. Some IgGs have been found to crosslink target receptors and thereby mimic the effect of the natural ligand. Cross-linking can lead to receptor activation (e.g. receptor phosphorylation). In contrast, monovalent Fab's derived from the same antibody do not lead to receptor cross-linking and, if the appropriate epitope is targeted, prevent the natural ligand from binding. Thus, while the IgG acts agonistic, the Fab fragment of the same antibody acts antagonistic. Examples are the insulin receptor (Kahn et al., Proc Natl Acad Sci USA. (1978) 75:4209-13), the EGF receptor (Schreiber et al., J Biol Chem. (1983) 258:846-53), the EPO receptor (Schneider et al, Blood (1997) 89:473-82), the GH receptor (Wan et al., Mol Endocrinol. (2003) 17:2240-50) or the beta2-Adrenoceptor (Mijares et al., Mol Pharmacol. (2000) 58:373-9).

However, constructs with such monovalent properties, e.g. Fab fragments, may be disadvantageous, due to other properties, such as a short half-life in vivo. It would, therefore, be highly desirable to combine a monovalent antibody-antigen interaction with the properties of a full-length immunoglobulin comprising the Fc domain. Such molecules are provided in the present invention.

### Summary of the invention

It is an object of the present invention to provide proteinaceous binding molecules, such as immunoglobulins, which are monovalent in respect of binding to the target molecule, but which retain the properties of a full length immunoglobulin in respect of size and presence of the Fc domain.

It is another object of the invention to provide monovalent proteinaceous binding molecules, in particular full-length immunoglobulins which are easy to purify from the culture supernatant of respective production cell lines.

It is another object of the present invention to provide a method to purify the proteinaceous binding molecules of to the present invention by way of making use of a purification tag for purification procedures, such as binding of the proteinaceous binding molecules to a matrix or resin with affinity for the purification tag.

Proteinaceous binding molecules according to the present invention are generated by introducing a purification tag into one variable domain of a proteinaceous binding molecule, such as an full-length immunoglobulin. Preferably the purification tag is introduced into the variable heavy chain of one arm of the immunoglobulin, most preferably into the H-CDR3 region or, alternatively, into the H-CDR2 region. Introduction of the purification tag leads to a loss of the specificity of the respective arm of the immunoglobulin.

Proteinaceous binding molecules of the present invention can be purified conveniently from the culture supernatant of respective production cell lines, e.g. via affinity chromatography using a matrix with affinity for the purification tag.

In is an alternative object the present invention to provide proteinaceous binding molecules comprising at least two variable domains, wherein one variable domain binds to a target molecule, and wherein the other variable domains are inert. Such proteinaceous binding molecules are monovalent in respect of binding to the target molecule, but retain the properties of a full length immunoglobulin in respect of size and presence of the Fc domain.

Proteinaceous binding molecules of the present invention have numerous uses in the diagnosis and treatment of diseases and disorders, such as cancer, immunological diseases or inflammatory diseases.

### Brief description of the figures

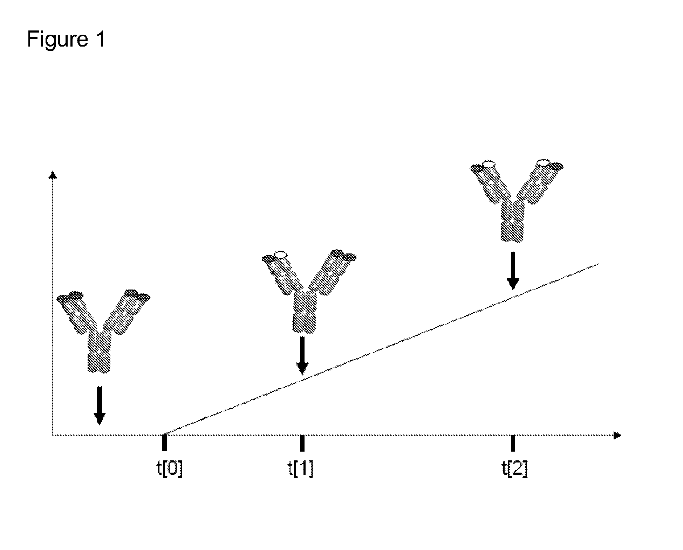

Figure 1 depicts the purification of proteinaceous binding molecules of the present invention via Ni-NTA chromatography.

The x-axis is the time axis. The y-axis indicates the concentration of the ingredient used to elute the material from the column (in the present case imidazole). 'Filled circles' on the antibodies indicate, that the respective CDRs are specific for a target molecule. 'Open circles' indicate that the respective CDRs comprise a purification tag. The three different immunoglobulin species elute at different fractions in an imidazole gradient.

### Detailed description of the invention

As used herein, a molecule, such as a proteinaceous binding molecule, "binds specifically to," is "specific to/for" or "specifically recognizes" a target molecule, such as an antigen, if such proteinaceous binding molecule is able to discriminate between such target molecule and one or more reference molecules(s), since binding specificity is not an absolute, but a relative property. In its most general form (and when no defined reference is mentioned), "specific binding" is referring to the ability of the proteinaceous binding molecule to discriminate between the target molecules of interest and an unrelated molecule, as determined, for example, in accordance with one of the following methods. Such methods comprise, but are not limited to Western blots, ELISA-, RIA-, ECL-, IRMA-tests and peptide scans. For example, a standard ELISA assay can be carried out. The scoring may be carried out by standard color development (e.g. secondary antibody with horseradish peroxide and tetramethyl benzidine with hydrogenperoxide). The reaction in certain wells is scored by the optical density, for example, at 450 nm. Typical background (=negative reaction) may be 0.1 OD; typical positive reaction may be 1 OD. This means the difference positive/negative can be more than 10-fold. Typically, determination of binding specificity is performed by using not a single reference molecule, but a set of about three to five unrelated molecules, such as milk powder, BSA, transferrin or the like.

However, "specific binding" also may refer to the ability of a proteinaceous binding molecule to discriminate between the target molecule and one or more closely related molecule(s), which are used as reference points, e.g. between target X and target Y. Additionally, "specific binding" may relate to the ability of a proteinaceous binding molecule to discriminate between different parts of its target molecule, e.g. different domains or regions of the target molecule, such as epitopes in the N-terminal or in the C-terminal region of the target molecule, or between one or more key amino acid residues or stretches of amino acid residues of the target molecule.

The term "proteinaceous binding molecule" as used herein refers to a molecule comprising at least two amino-acids in peptidic linkage with each other. The proteinaceous molecule is preferably a molecule that is produced by a biological organism or a part, derivative and/or analogue thereof. It is clear that derivatives generated through modification of the compound after the production by a biological molecule are also preferred compound of the invention. In a particularly preferred embodiment the proteinaceous binding molecules are immunoglubulins, such as antibodies, or fragments thereof. Other preferred proteinaceous binding molecules include scaffolds with antibody-like properties, such as affibodies (which comprise the Z-domain of protein A), immunity proteins (such as ImmE7), cytochrome b₅₆₂, proteins comprising ankyrin repeats, PDZ domains or Kunitz domains, insect defensin A, scorpion toxins (such as charybdotoxin or CTLA-4), knottins (such as Min-23, neocarzinostatin, CBM4-2 or tendamistat), anticalins or armadillo repeat proteins.

An "immunoglobulin" (Ig), as used herein, refers to a protein belonging to the class IgG, IgM, IgE, IgA, or IgD (or any subclass thereof), and includes all conventionally known antibodies and functional fragments thereof. A "functional fragment" of an antibody/immunoglobulin as defined herein refers to a fragment of an antibody/immunoglobulin (e.g., a variable region of an IgG) that comprises at least two variable domains in which the first variable domains retains the binding properties of the respective full-length immunoglobulin to the target molecule. Functional fragments of the invention include F(ab')2 fragments or Fab-dHLX fragments. Such fragments may be engineered to minimize or completely remove the intermolecular disulphide interactions that occur between the CH1 and CL domains. The term "functional fragment" of a proteinaceous binding molecule of the present invention refers to any proteinaceous moiety, in which wherein a first variable domain, but none of said other variable domains, binds to a target molecule of interest.

The term "variable domain" refers to the portion of a proteinaceous binding molecule which differs extensively in sequence between different proteinaceous binding molecules, and which confers binding and specificity of the proteinaceous binding molecule to a target molecule of interest (or lack thereof). In accordance with the present invention, peptide sequences, in particular purification tags as defined herein, are introduced into variable domains in a manner such that a variable domain does no longer specifically bind to a target molecule. Variable domains may have any shape or form. They may consist of polypeptide stretches comprised in one, two, or even more than two polypeptide chains. A variable domain of an immunoglobulin, such as an antibody, or a functional fragment thereof, typically comprises the entire antigen-binding region of one arm of the immunoglobulin molecule. It includes the CDR regions of both variable chains of one arm of the immunoglobulin molecule, i.e. the H-CDR3, the H-CDR2, the H-CDR1, the L-CDR3, the L-CDR2 and the L-CDR1, and the adjacent framework regions required for specific binding to a given target molecule.

An "antigen-binding region" of an antibody typically is found in one or more hypervariable region(s) of an antibody, i.e., the CDR-1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320). A preferred class of immunoglobulins for use in the present invention is IgG.

According to the present invention, the first variable domain of a proteinaceous binding molecule binds to a target molecule. The other variable domains of the proteinaceous binding molecule have no binding specificity to the same target molecule. The proteinaceous binding molecule is, therefore, monovalent in respect of binding to the target molecule. Furthermore, at least one of the other variable domains, i.e. the variable domains which do not bind to the target molecule, comprises a purification tag suitable for purification of said proteinaceous binding molecule.

An antibody of the invention may be derived from a recombinant antibody library that is based on amino acid sequences that have been designed in silico and encoded by nucleic acids that are synthetically created. In silico design of an antibody sequence is achieved, for example, by analyzing a database of human sequences and devising a polypeptide sequence utilizing the data obtained therefrom. Methods for designing and obtaining in silico-created sequences are described, for example, in Knappik et al., J. Mol. Biol. (2000) 296:57; Krebs et al., J. Immunol. Methods. (2001) 254:67; Rothe et al., J. Mol. Biol (2008) in press, and U.S. Patent No. 6,300,064 issued to Knappik et al., which hereby are incorporated by reference in their entirety.

"Target molecule" as refered to herein refers to any molecule of interest that binds to the first variable domain of the proteinaceous binding molecule. The target molecule refers to the molecule to which binding of the proteinaceous binding molecule is desired in order to achieve a certain biological effect and is to be discriminated from the binding partner of the second, or any subsequent, variable domain which comprises the purification tag. Typically and preferably the target molecule is a peptide, a protein or any other proteinaceous molecule. Alternatively the target molecules may be any other organic or inorganic molecules, such as carbohydrates, fatty acids, lipids, dyes or flourophors.

The term "purification tag" as used herein refers to any peptide sequence suitable for purification or identification of a proteinaceous binding molecule. The purification tag specifically binds to another moiety with affinity for the purification tag. Such moieties which specifically bind to a purification tag are usually attached to a matrix or a resin, such as agarose beads. Moieties which specifically bind to purification tags include antibodies, nickel or cobalt ions or resins, biotin, amylose, maltose, and cyclodextrin. Exemplary purification tags include histidine tags (such as a hexahistidine peptide), which will bind to metal ions such as nickel or cobalt ions. Other exemplary purification tags are the myc tag (EQKLISEEDL), the Strep tag (WSHPQFEK), the Flag tag (DYKDDDDK) and the V5 tag (GKPIPNPLLGLDST). The term "purification tag" also includes "epitope tags", i.e. peptide sequences which are specifically recognized by antibodies. Exemplary epitope tags include the FLAG tag, which is specifically recognized by a monoclonal anti-FLAG antibody. The peptide sequence recognized by the anti-FLAG antibody consists of the sequence DYKDDDDK or a substantially identical variant thereof. The term "purification tag" also includes substantially identical variants of purification tags. "Substantially identical variant" as used herein refers to derivatives or fragments of purification tags which are modified compared to the original purification tag (e.g. via amino acid substitutions, deletions or insertions), but which retain the property of the purification tag of specifically binding to a moiety which specifically recognizes the purification tag.

In certain embodiments the present invention relates to proteinaceous binding molecules comprising at least two variable domains, wherein one variable domain binds to a target molecule, and wherein the other variable domains are inert.

"Inert" as used herein in the context of the binding specificity of a variable domain refers to a variable domain which does not have binding specificity to another molecule. In particular, an inert variable domain does not bind to a target molecule or any other molecule, and it does not comprise a purification tag. Proteinaceous binding molecules comprising at least two variable domains, wherein one variable domain binds to a target molecule, and wherein the other variable domains are inert, possess monovalent binding specificity for a target molecule and may be used in accordance with the present invention.

The present invention also relates to the DNA molecules that encode the proteinaceous binding molecules of the invention. DNA molecules of the invention are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 (Sambrook, J., Fritsch, E. F. and Maniatis, T. (1989) Molecular Cloning: A laboratory manual, Cold Spring Harbor Laboratory Press, Cold Spring Harbor, USA) and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

The present invention further provides recombinant DNA constructs comprising one or more of the nucleotide sequences of the present invention. A recombinant construct of the present invention can be used in connection with a vector, such as a plasmid, phagemid, phage or viral vector, into which a DNA molecule encoding a proteinaceous binding molecule of the invention is inserted. The encoded gene may be produced by techniques described in Sambrook et al., 1989, and Ausubel et al., 1989. Alternatively, the DNA sequences may be chemically synthesized using, for example, synthesizers. See, for example, the techniques described in Oligonucleotide Synthesis (1984, Gait, ed., IRL Press, Oxford), which is incorporated by reference herein in its entirety. Recombinant constructs of the invention are comprised with expression vectors that are capable of expressing the RNA and/or protein products of the encoded DNA(s). The vector may further comprise regulatory sequences, including a promoter operably linked to the open reading frame (ORF). The vector may further comprise a selectable marker sequence. Specific initiation and bacterial secretory signals also may be required for efficient translation of inserted target gene coding sequences.

The present invention further provides host cells containing at least one of the DNA constructs of the present invention. The host cell can be virtually any cell for which expression vectors are available. It may be, for example, a higher eukaryotic host cell, such as a mammalian cell, a lower eukaryotic host cell, such as a yeast cell, and may be a prokaryotic cell, such as a bacterial cell. Introduction of the recombinant construct into the host cell can be effected, for example, by calcium phosphate transfection, DEAE, dextran mediated transfection, electroporation or phage infection.

In certain embodiments, the present invention provides proteinaceous binding molecules comprising at least two variable domains, wherein one variable domain binds to a target molecule, and wherein at least one other variable domain comprises a purification tag. The proteinaceous binding molecule may comprise two, three four or more than four variable domains. In preferred embodiments, the proteinaceous binding molecule comprises two variable domains.

According to the present invention, one variable domain of the proteinaceous binding molecule binds to a target molecule. None of the other variable domains binds to the target molecule, in particular the variable domain comprising the purification tag. In certain embodiments, the present invention relates to proteinaceous binding molecules comprising at least two variable domains, wherein the first variable domain binds to a target molecule and wherein at least one other variable domain comprises a purification tag suitable for purification of said proteinaceous binding molecule, and wherein none of said other variable domain binds to said target molecule. In other embodiments the proteinaceous binding molecule comprises at least two variable domains wherein the first variable domain binds to a target molecule, but none of said other variable domain binds to said target molecule.

In certain embodiments, the purification tag is selected from a histidine tag, a Strep tag, a myc tag, a V5 tag and a Flag tag, or a substantially identical variant thereof. In preferred embodiments, the purification tag is a histidine tag (His tag), most preferably a hexahistidine peptide. In alternative embodiments, the purification tag is a substantially identical variant of a histidine tag, such as a pentahistidine peptide or a heptahistine peptide. Other histidine tags with even more histidine residues, such as eight, nine, ten, or even more histidine residues, or polyhistidine stretches, in which one or more histidine residue is substituted to another amino acid, but which is still able to bind to Ni-NTA, may be used in accordance with the present invention as well.

In certain embodiments, the proteinaceous binding molecule is an immunoglobulin or a functional fragment thereof. In preferred embodiments, said immunoglobulin is an antibody, more preferably a full-length antibody.

In certain embodiments, the immunoglobulin is a human immunoglobulin. In alternative embodiments said immunoglobulin is a immunoglobulin from a rodent, such as a mouse or a rat.

In certain embodiments, the immunoglobulin is of any one of the classes IgG, IgM, IgE, IgA, and IgD. In preferred embodiments, the immunoglobulin is of the class IgG. In certain embodiments, the immunoglobulin is a human immunoglobulin of any one of the classes IgG, IgM, IgE, IgA, and IgD. In preferred embodiments, the immunoglobulin is a human immunoglobulin of the class IgG.

In certain embodiments, the immunoglobulin is of any one of the subclasses IgG1, IgG2, IgG3 and IgG4. In preferred embodiments, the immunoglobulin is of the subclass IgG1. In certain embodiments, the immunoglobulin is a human immunoglobulin of any one of the subclasses IgG1, IgG2, IgG3 and IgG4. In preferred embodiments, the immunoglobulin is a human immunoglobulin of the subclass IgG1.

In preferred embodiments the proteinaceous binding molecule is an immunoglobulin. In these embodiments the variable domains comprise a variable heavy (VH) chain and a variable light (VL) chain. In preferred embodiments the proteinaceous binding molecule is an immunoglobulin comprising two variable domains, each variable domain comprising a variable heavy (VH) chain and a variable light (VL) chain.

In certain embodiments, the variable heavy (VH) chain comprises three complementary determining regions, H-CDRs. In preferred embodiments the proteinaceous binding molecule is an immunoglobulin comprising a variable heavy (VH) chain comprisings three complementary determining regions, H-CDR1, H-CDR2 and H-CDR3.

In certain embodiments, the variable light (VH) chain comprises three complementary determining regions, L-CDRs. In preferred embodiments the proteinaceous binding molecule is an immunoglobulin comprising a variable light (VH) chain comprisings three complementary determining regions, L-CDR1, L-CDR2 and L-CDR3.

In certain embodiments, the purification tag is comprised in the variable heavy (VH) chain or the variable light (VL) chain of the proteinaceous binding molecule or immunoglobulin. In preferred embodiments, said purification tag is comprised in the variable heavy (VH) chain of the proteinaceous binding molecule or immunoglobulin. In more preferred embodiments the purification tag is comprised in any one of the three complementary determining regions (H-CDRs) of the variable heavy (VH) chain. In even more preferred embodiments the purification tag is comprised in the H-CDR3, the H-CDR2 or the H-CDR1, more preferrably the H-CDR3 or the H-CDR2 and most preferrably the H-CDR3.

In certain embodiments, the purification tag is comprised in the variable light (VL) chain of the proteinaceous binding molecule or immunoglobulin. In more preferred embodiments, the purification tag is comprised in any one of the three complementary determining regions (L-CDRs) of the variable light (VL) chain. In even more preferred embodiments, the purification tag is comprised in the L-CDR3, the L-CDR2 or the L-CDR1, and in most preferred embodiments in the L-CDR3.

The present invention further provides nucleic acid sequences that encode the proteinaceous binding molecules of the present invention. Proteinaceous binding molecules of the present invention may consist of one, two, three four, or even more polypeptides. Each of said polypeptides may be encoded by the same or by different nucleic acids. Likewise, the nucleic acids encoding said individual peptides of a proteinaceous binding molecule of the present invention may be present on the same or on different vectors.

The present invention further provides nucleic acid sequences that encode a variable domain comprising a purification tag. Preferably, said variable domain is comprised in an immunoglobulin or a functional fragment thereof. In certain embodiments, said variable domain is comprised in the variable heavy (VH) chain or the variable light (VL) chain of said immunoglobulin, preferably in the variable heavy (VH) chain, and more preferably in the H-CDR3 of said variable heavy (VH) chain.

The present invention further provides vectors comprising the nucleic acid sequences of the proteinaceous binding molecules or comprising the nucleic acid sequences of variable domains comprising a purification tag according to the present invention.

The present invention further provides cells and host cells comprising said vectors. Preferably said cells or host cells are mammalian cells. Also preferably said cells or host cells are isolated cells or isolated host cells.

The present invention further provides pharmaceutical compositions comprising a proteinaceous binding molecule of the present invention and a pharmaceutical acceptable carrier or excipient therefor.

The present invention further provides therapeutic methods for treating a disease or disorder comprising the step of administering to a subject in need thereof a therapeutically effective amount of the pharmaceutical composition comprising a proteinaceous binding molecule of the present invention and a pharmaceutical acceptable carrier or excipient therefor. Preferably said disease or disorder is associated with the undesired presence of the target molecule.

A "therapeutically effective" amount hereby is defined as the amount of a proteinaceous binding molecule that alone, or in combination with other therapies, provides a therapeutic benefit in the treatment or management of an adverse condition. The term can encompass an amount that improves overall therapy, reduces or avoids unwanted effects, or enhances the therapeutic efficacy of or synergies with another therapeutic agent. The "subject" in need of a treatment may be a human or non-human animal (e.g., rabbit, rat, mouse, monkey or other lower-order primate).

A proteinaceous binding molecule of the invention might be co-administered with known medicaments, and in some instances the proteinaceous binding molecule might itself be modified. For example, a proteinaceous binding molecule could be conjugated to an immunotoxin or radioisotope to potentially further increase efficacy.

The proteinaceous binding molecules can be used as a therapeutic or a diagnostic tool in a variety of situations where the target molecule is undesirably expressed or found. To treat any diseases or disorders, pharmaceutical compositions for use in accordance with the present invention may be formulated in a conventional manner using one or more physiologically acceptable carriers or excipients. A proteinaceous binding molecule of the invention can be administered by any suitable means, which can vary, depending on the type of disorder being treated. Possible administration routes include parenteral (e.g., intramuscular, intravenous, intraarterial, intraperitoneal, or subcutaneous), intrapulmonary and intranasal, and, if desired for local immunosuppressive treatment, intralesional administration. In addition, a proteinaceous binding molecule of the invention might be administered by pulse infusion, with, e.g., declining doses of the proteinaceous binding molecule. Preferably, the dosing is given by injections, most preferably intravenous or subcutaneous injections, depending in part on whether the administration is brief or chronic. The amount to be administered will depend on a variety of factors such as the clinical symptoms, weight of the individual, whether other drugs are administered. The skilled artisan will recognize that the route of administration will vary depending on the disorder or condition to be treated.

Determining a therapeutically effective amount of the proteinaceous binding molecule, according to this invention, largely will depend on particular patient characteristics, route of administration, and the nature of the disorder being treated. General guidance can be found, for example, in the publications of the International Conference on Harmonisation and in Remington's Pharmaceutical Sciences, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references. Efficacy may be determined utilizing the same guidance.

The present invention further provides methods for detecting a target molecule in a subject or a sample, comprising the step of contacting said subject or sample with a proteinaceous binding molecule of the present invention.

The present invention further provides methods for diagnosing a disease or disorder in a subject, comprising the step of contacting said subject or sample with a proteinaceous binding molecule of the present invention.

The present invention further provides the use of a proteinaceous binding molecule of the present invention for the manufacture of a medicament.

The present invention further provides a proteinaceous binding molecule of the present invention for the treatment of a disease or disorder.

The present invention further provides the use of a proteinaceous binding molecule of the present invention for the diagnosis of a disease or disorder.

The present invention further provides the use of a proteinaceous binding molecule of the present invention for the detection of a target molecule.

In certain embodiments the proteinaceous binding molecule is an immunoglobulin, such as an antibody. In such cases, the cell line transfected with the nucleic acid molecules of the present invention produces two different variable heavy (VH) chains - one VH chain which is part of the variable domain which binds to the target molecule (hereinafter "VH-targ"), and one VH chain which is part of the variable domain which comprises the purification tag (hereinafter "VH-puri"; alternatively this VH chain may be inert). Only one variable light (VL) chain is produced. This leads to the production of three different immunoglobulin species:
- Species A comprises two variable heavy (VH) chains of the type VH-targ.
- Species B comprises one variable heavy (VH) chain of the type VH-targ and one variable heavy (VH) chain of the type VH-puri.
- Species C comprises two variable heavy (VH) chains of the type VH-puri.
   All immunoglobulin species comprise two identical variable light (VL) chains.

Species A of the immunoglobulins described above does not comprise a purification tag. Species B comprises one purification tag, species C comprises two purification tags. Species B is the species with the desired properties according to the present invention, i.e. monovalent binding of the target molecule.

Species B and C can both be purified via a moiety which has affinity for the purification tag, e.g. via Ni-NTA, if the purification tag is a His tag. Since the avidity of immunoglobulin species C is much higher compared to species B due to the presence of a second purification tag, these species can be conveniently separated via appropriate purification steps. Possible purification techniques include chromatography, such as column chromatography. Elution can be accomplished by various ways. Preferably a gradient is applied. Respective techniques are known to the skilled artisan. If a histidine tag is used, elution is most conveniently achieved by way of a imidazole gradient. The process is represented in Figure 1. Due to its higher avidity, species C has higher affinity to the column, and hence higher concentrations of a solution, such as a solution comprising imidazole, will be needed to recover said species from the chromatography column. By way of this technique, i.e. a higher imidazole concentration for elution, species B can be conveniently separated from species C.

Therefore, the present invention provides a method to purify a proteinaceous binding molecule of the present invention, comprising the steps of (a) providing a solution comprising a proteinaceous binding molecule of the present invention, (b) bringing the solution of step (a) in contact with a moiety with affinity to the purification tag of said proteinaceous binding molecule, (c) allowing the binding of the proteinaceous binding molecule to said moiety, (d) removing all or substantially all constituents of the solution which do not bind to the moiety, and (e) recovering the proteinaceous binding molecule from the moiety.

The solution comprising a proteinaceous binding molecule of the present invention is preferably the culture supernatant of a cell expressing said proteinaceous binding molecule. Alternatively, any other solution comprising a proteinaceous binding molecule according to the present invention may be used.

The solution comprising a proteinaceous binding molecule of the present invention may further comprise a proteinaceous binding molecules comprising two purification tags.

Preferably, the proteinaceous binding molecule of the present invention is separated from a solution comprising proteinaceous binding molecules comprising two purification tags via a gradient, preferably a imidazole gradient.

In certain embodiments, the present invention provides a method to separate a first proteinaceous binding molecule comprising at least two variable domains, wherein one variable domain binds to a target molecule, and wherein one other variable domain comprises a purification tag from a second proteinaceous binding molecule comprising two purification tags, comprising the steps of (a) providing a solution comprising a the first and the second proteinaceous binding molecule, (b) bringing the solution of step (a) in contact with a moiety with affinity to the purification tag of said proteinaceous binding molecules, (c) allowing the binding of the proteinaceous binding molecules to said moiety, (d) removing all or substantially all constituents of the solution which do not bind to the moiety, and (e) recovering the proteinaceous binding molecules from the moiety in a manner that the first proteinaceous binding molecule is separated from the second proteinaceous binding molecule. In preferred embodiments step (e) comprises the application of a gradient to recover the proteinaceous binding molecule from the moiety with affinity for the purification tag. If the purification tag is a histidine tag, then an imidazole gradient is preferred.

### Examples

### Example 1: Generation of Fab fragments containing a purification tag in the H-CDR3 region

MOR03207_FS is a Fab with specificity for hen egg lysozyme. It comprises a Flag tag and a Strep tag at the C-terminus of the VH chain, i.e. outside the variable domain. Via appropriate primer design and conventional cloning techniques, a hexahistidine tag is introduced into the H-CDR3 of the variable heavy chain of MOR03207_FS, yielding MOR0xxxx. MOR0xxxx comprises a Flag tag and a Strep tag.

Test purifications confirm that both MOR03207_FS and MOR0xxxx can be purified via the Strep tag. However, only MOR0xxxx, but not MOR03207_FS, can be purified by Ni-NTA chromatography, confirming that the hexahistidine tag is functional. Finally, via FACS analysis and ELISA tests it is confirmed that MOR03207_FS, but not MOR0xxxx, is able to bind to hen egg lysozyme, indicating that the specificity of the original Fab fragment was destroyed by way of incorporation of the hexahistidine tag.

Next, the VH of MOR0xxxx was combined by cloning with the VL of MOR03080. MOR03080 is a Fab with specificity for human CD38. The yielding construct, designated MOR0zzzz, hence contains the VH of MOR0xxxx and the VL of MOR03080.

Via FACS analysis and ELISA tests it is confirmed that MOR03080, but not MOR0zzzz, is able to bind to human CD38. Likewise, MOR03207_FS, but not MOR0zzzz, is able to bind to hen egg lysozyme. Finally, MOR0xxxx and MOR0zzzz, but not MOR03207_FS and MOR03080, can be purified by Ni-NTA chromatography.

In summary, it is demonstrated that via the incorporation of a purification tag, such as a hexahistidine tag, into the variable domain of a proteinaceous binding molecule, such as a H-CDR3, it is possible to generate binders which (a) can be easily purified by way of their binding to respective affinity resins, and (b) are no longer able to bind to the target molecule of the original binder.

### Example 2: Generation of full-length IgG's containing a purification tag in the H-CDR3 region

Monovalent immunoglobulins of the present invention can be generated as described in this example. Alternatively, other techniques may be employed. For example certain experimental techniques exist, which allow the favourable pairing of the desired immunoglobulin heavy chains. For example, bispecific IgG's can be generated by a technology using engineered disulfide bonds and "knob into holes" mutations (Merchant et al, Nature Biotechnology (1998) 16, 677), enabling the efficient pairing of the desired heavy chains. Other related techniques known in the art may be employed as well.

In the following, Fab fragments of Example 1 are converted into IgG1 format. Therefore, the respective constructs are cloned into appropriate vectors.

Variable heavy chains of MOR0xxxx and MOR03080 are cloned into pMorph2_hulgG1, a vector harbouring the constant human gamma1 constant region, thereby generating vectors pMorph2_hulgG1_MOR0xxxx and pMorph2_hulgG1_MOR03080, respectively. The variable light chain of of MOR03080 is cloned into pMorph2_hu_lambda, a vector harbouring the constant human lambda light chain, yielding in vector pMorph2_hu_lambda_MOR03080. All three constructs are co-transfected and expressed in a human cell line, such as HKB11 (ATCC number: CRL-12568; J Biomed Sci (2002) 9, 631. The cell culture supernatant is used for further experiments. Optionally the cell culture supernatant is prepurifed via a Protein A column.

Since two different heavy chains are produced in the transfected cell line, altogether three different immunoglobulin species are produced. One species contains two heavy chains of MOR03080. This immunoglobulin species does not contain a hexahistine tag and hence has no affinity to Ni-NTA and monovalent specificity foe CD38. The second immunoglobulin species contains one heavy chain of MOR03080 and one heavy chain of MOR0xxxx. This immunoglobulin species is the species with the desired properties according to the present invention. It has affinity to Ni-NTA. Finally, the third immunoglobulin species contains two heavy chains of MOR0xxxx. This species has no affinity to the target molecule (in this case human CD38). However, this species contains a hexahistidine tag on both arms of the immunoglobulin and therefore shows bivalent binding of Ni-NTA, hence having higher avidity for Ni-NTA. Since the avidity to Ni-NTA of this species is much higher than the affinity of the second, desired, immunoglobulin species, it can be conveniently separated via Ni-NTA chromatography, e.g. via a imidazole gradient. This elution process is represented in Figure 1.

In summary, full-length immunoglobulins with monovalent affinity for a target molecule are produced. These molecules can be easily produced and purified via Ni-NTA chromatography employing the purification tag.

## Claims

1. A proteinaceous binding molecule comprising at least two variable domains, wherein one variable domain binds to a target molecule, and wherein at least one other variable domain comprises a purification tag or is inert.

2. The proteinaceous binding molecule of claim 1, wherein the proteinaceous binding molecule comprises two variable domains.

3. The proteinaceous binding molecule of claim 1 or 2, wherein the variable domain comprising the purification tag does not bind to the target molecule.

4. The proteinaceous binding molecule of any one of claims 1 to 3, wherein said purification tag is selected from a histidine tag, a Strep tag, a myc tag, a Flag tag and a V5 tag, or a substantially identical variant thereof.

5. The proteinaceous binding molecule of claim 4, wherein said purification tag is a histidine tag or a substantially identical variant thereof.

6. The proteinaceous binding molecule of claim 5, wherein said histidine tag is a hexahistidine peptide.

7. The proteinaceous binding molecule of any one of claim 1 to 6, wherein said proteinaceous binding molecule is an immunoglobuin or a functional fragment thereof.

8. The proteinaceous binding molecule of claim 7, wherein said immunoglobulin is an antibody.

9. The proteinaceous binding molecule of any one of claims 7 or 8, wherein said immunoglobuilin is of the class IgG.

10. The proteinaceous binding molecule of claim 9, wherein said immunoglobulin is of any one of the subclasses IgG1, IgG2, IgG3 and IgG4.

11. The proteinaceous binding molecule of claim 10, wherein said immunoglobulin is of the subclass IgG1.

12. The proteinaceous binding molecule according to any one of the preceding claims, wherein each of said variable domains comprises a variable heavy (VH) chain and a variable light (VL) chain.

13. The proteinaceous binding molecule of claim 12, wherein said variable heavy (VH) chain comprises three complementary determining regions (H-CDRs).

14. The proteinaceous binding molecule of claim 12, wherein said variable light (VL) chain comprises three complementary determining regions (L-CDRs).

15. The proteinaceous binding molecule according to any one of claims 12 to 14, wherein said purification tag is comprised in the variable heavy (VH) chain or the variable light (VL) chain.

16. The proteinaceous binding molecule according to claim 15, wherein said purification tag is comprised in the variable heavy (VH) chain.

17. The proteinaceous binding molecule according to claim 16, wherein said purification tag is comprised in any one of the three complementary determining regions (H-CDRs) of the variable heavy (VH) chain.

18. The proteinaceous binding molecule according to claim 17, wherein said purification tag is comprised in the H-CDR3 or the H-CDR2.

19. The proteinaceous binding molecule according to claim 18, wherein said purification tag is comprised in the H-CDR3.

20. The proteinaceous binding molecule according to claim 15, wherein said purification tag is comprised in the variable light (VL) chain.

21. The proteinaceous binding molecule according to claim 20, wherein said purification tag is comprised in any one of the three complementary determining regions (L-CDRs) of the variable light (VH) chain.

22. The proteinaceous binding molecule according to claim 21, wherein said purification tag is comprised in the L-CDR2.

23. A nucleic acid sequence that encodes a proteinaceous binding molecule of any one of the preceding claims.

24. An nucleic acid sequence that encodes a variable domain comprising a purification tag.

25. The nucleic acid sequence of claim 24, wherein said variable domain is comprised in an immunoglobulin or a functional fragment thereof.

26. The nucleic acid of claim 25, wherein said variable domain is comprised in the variable heavy (VH) chain or the variable light (VL) chain of said immunoglobulin.

27. The nucleic acid of claim 26, wherein said variable domain is comprised in the variable heavy (VH) chain of said immunoglobulin.

28. The nucleic acid of claim 27, wherein said variable domain is comprised in the H-CDR3 of said variable heavy (VH) chain.

29. A vector comprising a nucleic acid sequence of any one of claims 23 to 28.

30. A cell comprising a vector according to claim 29.

31. A cell according to claim 30, wherein said cell is a mammalian cell.

32. The cell according to claim 30 or 31, wherein said cell is an isolated cell.

33. A pharmaceutical composition comprising a proteinaceous binding molecule of any one of claims 1 to 22 and a pharmaceutical acceptable carrier or excipient therefor.

34. A method for treating a disease or disorder, comprising the step of administering to a subject in need thereof a therapeutically effective amount of the pharmaceutical composition of claim 33.

35. The method according to claim 34, wherein said disease or disorder is associated with the undesired presence of the target molecule.

36. A method for detecting a target molecule in a subject or a sample, comprising the step of contacting said subject or sample with a proteinaceous binding molecule of any one of claims 1 to 22.

37. A method for diagnosing a disease or disorder in a subject, comprising the step of contacting said subject or sample with a proteinaceous binding molecule of any one of claims 1 to 22.

38. Use of a proteinaceous binding molecule according to of any one of claims 1 to 22 for the manufacture of a medicament.

39. A proteinaceous binding molecule according to of any one of claims 1 to 22 for the treatment of a disease or disorder.

40. Use of a proteinaceous binding molecule according to any one of claims 1 to 22 for the diagnosis of a disease or disorder.

41. Use of a proteinaceous binding molecule according to any one of claims 1 to 22 for the detection of a target molecule.

42. A method to purify a proteinaceous binding molecule comprising the steps of (a) providing a solution comprising a proteinaceous binding molecule according to any one of claims 1 to 22, (b) bringing the solution of step (a) in contact with a moiety with affinity to the purification tag of said proteinaceous binding molecule, (c) allowing the binding of the proteinaceous binding molecule to said moiety, (d) removing all or substantially all constituents of the solution which do not bind to the moiety, and (e) recovering the proteinaceous binding molecule from the moiety.

43. The method according to claim 42, wherein said solution further contains a proteinaceous binding molecules comprising two purification tags.

44. The method according to claim 43, wherein the proteinaceous binding molecule according to any one of claims 1 to 20 is separated from the proteinaceous binding molecules comprising two purification tags via a gradient.

45. The method according to claim 44, wherein said gradient is an imidazole gradient.
